# EUROPEAN PATENT APPLICATION

(11) **EP 2 412 793 A1**
(43) Date of publication of application: **01.02.2012**
(21) Application number: 10171529.0
(22) Date of filing: 30.07.2010
(51) Int. Cl.: C12M 1/00

(54) **Circulatory photobioreactor**

(71) Applicant: Kairos Global Co., Ltd., Seoul 135-010 (KR); Eulgi University Industry Academy Corporation Foundation, Seongnam-si Gyeonggi-do 461-713 (KR)
(72) Inventor: Kim, Kwang Ho, Dangjin-gun, Chungcheongnam-do 343-882, (KR); Kang, Hee-Gyoo, Sanggye-9-dong, Nowon-gu, Seoul 139-763, (KR); Kwon, Young II, Seo-gu, Daejeon-si 302-845. (KR); Kim, Sun Jong, Anyang-si, Gyeonggi-do 431-774 (KR); Lim, Hee Joung, Songpa-gu, Seoul 138-942, (KR); Kim, Mi Jeong, Seongnam-si, Gyeonggi-do 462-832, (KR)
(74) Representative: Tomlinson, Kerry John

(57) **Abstract**

A circulatory photobioreactor is provided. The circulatory photobioreactor comprises a first cultivating part (100), a second cultivating part (300) and a pump part (200) connecting the first cultivating part and the second cultivating part. The first cultivating part comprises a culture tank (101) in which culture media are supplied and a first light source coupled to the culture tank, which illuminates the inside of the culture tank. The second cultivating part comprises a culture pipe (330) placed outside of the culture tank and is supplied with cultures from the culture tank and a second light source (320) coupled to the culture pipe, which illuminates the inside of the culture pipe. The pump part is connected to both the first cultivating part and the second cultivating part in order to circulate the culture solution between them.

## Description

### BACKGROUND OF THE INVENTION

### 1. Field of the Invention

The following description relates to a culture apparatus, particularly to a circulatory photobioreactor.

### 2. Description of the Related Art

Currently, crops cultivated world-wide are used as major food sources for humans. However, yield per acre is not too much and the energy efficiency of solar power is extremely low. On the contrary, photosynthetic microalgae capable of growing underwater with solar energy, carbon dioxide and minute amounts of inorganic salts are favorable, since they can be cultivated in areas in which crops do not grow, can provide at least 20 times more protein per acre than conventional crops and can provide various useful substances and rare natural substances which are not produced in microorganisms, animals or plants. In particular, since algae whose cell size is large precipitate well, isolating them and extracting some substances therefrom is easy. In addition, they use solar energy as a major energy source, thus it is possible to use solar energy efficiently. Further, since they use carbon dioxide as a carbon source and have a photosynthetic system producing oxygen as byproducts, they can alleviate air pollution.

Accordingly, photosynthetic microalgae including genus *Chlorella,* genus *Dunaliella* and genus *Spirulina* have been studied, particularly microalgae belonging to genus *Spirulina* (hereinafter referred to as "Spirulina") since it is bigger than other photosynthetic microalgae, grows easily in an alkali-contaminated environment and can be applied in food, pharmaceutical and other industries. In the meanwhile, temperate regions such as Korea having climate characteristics such as occurrence of four seasons, dramatic variations of seasonal temperature and sunshine are developing indoor culture technology rather than outdoor culture technology.

For examples, Korean Patent No. 235182 discloses a continuous culture apparatus comprising an automatic control heater, a culture vessel having an aeration apparatus, a frame having equipment for supporting the culture vessel, a medium-supplying pipe coupled to the side of the frame, a rubber tube for circulating culture solution and a water pump for circulating the culture solution. Korean Patent Gazette No. 2004-0019298 discloses an apparatus for cultivating microalgae comprising a culture vessel molded as a double cylinder-type consisting of an inner cylinder disposed horizontally and an outer cylinder, wherein at least the outer cylinder comprises a transparent material transmitting light and a gas inlet is opened at the bottom of the culture vessel. Korean Patent No. 679989 discloses a watercourse-type outdoor culture vessel for microalgae in which an inoculation culture vessel for cultivating inoculates is integrally installed. However, if one cultivates Spirulina using these apparatuses, the growing Spirulina attaches to the surface of the culture vessel excessively. Accordingly, the culture efficiency deteriorates and one can not obtain favorable cultural products owing to contamination by various germs.

In order to solve the above problems, a method comprising sealing a culture vessel and preventing contamination by various germs by filtering air supplied using a filter was used. For examples, Korean Patent Gazette No. 2002-0057882 discloses an outdoor large scale culture apparatus comprising an air filtering and UV sterilizing device, an air pump, a filtering device, a guide pipe, a filter, a needle valve and a culture vessel and Korean Gazette Patent No. 2002-0083558 discloses a high-density culturing apparatus comprising a culture vessel having a cover on the top side and a pH sensor and dispenser in the culture vessel, a frame for the culture vessel having fluorescent lights, a pH controller, an air pump and CO₂ culture tank.

### SUMMARY OF THE INVENTION

It is possible to prevent contamination by various germs using various apparatuses of the prior art. However, the problems of excessive photosynthetic microalgae attachment to the inner surface of the culture vessels and the deteriorating cultivation efficiency have not been solved until now. If the problems are solved, it is possible to improve the cultivation efficiency of photosynthetic microalgae remarkably. However, there is no report to teach such a solution. Thus, the purpose of the present invention is to provide a novel culture apparatus for cultivating microalgae capable of preventing attachment of photosynthetic microalgae to the inner surface of a culture vessel and thereby improving the cultivation efficiency.

The present inventors have solved the above-described problem and have confirmed that the area in which culture solution is exposed to light is maximized and growing photosynthetic microalgae do not attach to the inner surface of the culture vessel when the photosynthetic microalgae are cultivated in a circulating way.

In one aspect, the present invention provides a circulatory photosynthetic bioreactor comprising:
a first cultivating part comprising a culture tank in which culture media are supplied and a first light source coupled to the culture tank so as to illuminate the inside of the culture tank;
a second cultivating part comprising a culture pipe placed outside the culture tank and supplied with culture solution from the culture tank and a second light source coupled to the culture pipe so as to illuminate the inside of the culture pipe; and
a pump part connected to both the first cultivating part and the second cultivating part so as to circulate the culture solution between the first cultivating part and the second cultivating part.

In an embodiment, the first light source is placed inside the culture tank, but the invention is not limited thereto. In an embodiment, the second light source is coupled to the inner surface of the culture pipe or placed outside the culture pipe in the longitudinal direction of the culture pipe, but the invention is not limited thereto. In another embodiment, the culture pipe preferably has a parallel multiple folded form and comprises a plurality of second light sources coupled to the outside of the culture pipe in the longitudinal direction of the culture pipe, but the invention is not limited thereto. In a preferred embodiment, the culture pipe has an inside diameter of 3 to 30 cm. In a more preferred embodiment, the diameter is 5 to 20 cm. In a further preferred embodiment, the inside diameter is 10 to 20 cm. In the most preferred embodiment, the diameter is 10 to 15 cm. In a preferred embodiment, the first cultivating part further comprises at least one first culture solution inlet and at least one first culture solution outlet connected to both the culture tank and the pump part, and the second cultivating part further comprises at least one second culture solution inlet and at least one second culture solution outlet connected to the first culture solution outlet and to the second culture solution inlet, respectively, through the pump part, but the invention is not limited thereto. In another preferred embodiment, the first cultivating part further comprises a fresh media inlet for supplying fresh media into the culture tank and a final outlet for releasing culture solution from the culture tank, but the invention is not limited thereto, and the fresh media inlet preferably has a spray ball shape including a globular type end and a plurality of micro nozzles on the globular type end.

In an embodiment, the first cultivating part further comprises an agitator for mixing culture solution, which is attached to the inside of the culture tank. In another embodiment, the first cultivating part further comprises a pressure controlling valve for controlling pressure in the culture tank and the pressure controlling valve is preferably a one-way valve which opens by pressure of oxygen generated during the cultivation of photosynthetic microalgae.

In an embodiment, the first cultivating part further comprises a temperature controller for controlling temperature in the culture tank and the temperature controller is preferably a water-jacket coupled to the outside of the culture tank, but the invention is not limited thereto.

In an embodiment, the first cultivating part further comprises one or more ports for sensors coupled to the culture tank. In another embodiment, the first cultivating part further comprises a gas inlet coupled to the culture tank so as to inject gases into the culture tank.

In an embodiment, the second cultivating part further comprises an oxygen discharger and the oxygen discharger preferably comprises an earth sensor, a mass flow controller electrically connected to the earth sensor and a valve connected to the mass flow controller, but the invention is not limited thereto.

In a preferred embodiment, a flow rate controller is coupled to the pump part in order to control the flow rate of culture solution circulating through the second cultivating part. In a more preferred embodiment, the flow rate controller is built in the pump part.

In another aspect, the present invention provides a circulatory photosynthetic bioreactor comprising:
a first cultivating part comprising a culture tank in which culture media are supplied, a first media inlet, a first media outlet and a first light source each coupled to the culture tank;
a second cultivating part comprising a culture pipe arranged outside the culture tank in tubular shape and supplied with culture media from the culture tank, a second media inlet, a second media outlet and a second light source each coupled to the culture pipe, wherein the first media outlet is connected to the second media inlet and the first media inlet is connected to the second media outlet in order that the culture media can circulated between the first cultivating part and the second cultivating part.

The photosynthetic bioreactor may further comprise a pump part connecting the first cultivating part and the second cultivating part so as to circulate culture solution between the first cultivating part and the second cultivating part.

In a preferred embodiment, the pump part further comprises a flow rate controller electronically coupled to a pump thereof. In a more preferred embodiment, the flow rate controller is built in the pump part. In a preferred embodiment, the culture pipe has an inside diameter of 3 to 30 cm. In a more preferred embodiment, the diameter is 5 to 20 cm. In a further preferred embodiment, the inside diameter is 10 to 20 cm. In the most preferred embodiment, the diameter is 10 to 15 cm.

The circulatory photobioreactor of the present invention prevents attachment of photosynthetic microalgae on the inner surface of culture vessel and can increase the culture efficiency of the photosynthetic microalgae thereby. Accordingly, one can cultivate photosynthetic microalgae more economically.

In the description, "culture media" or "culture medium" means media for the cultivation without any living organism such as microalgae, whereas "culture solution" means a mixture of the culture media and microalgae to be cultivated.

### BRIEF DESCRIPTION OF THE DRAWINGS

The accompanying drawings, together with the specification, illustrate exemplary embodiments of the present invention, and, together with the description, serve to explain the principles of the present invention.

Fig. 1 is a schematic diagram showing a photobioreactor according to an embodiment of the present invention.

Fig. 2 is a sectional view of the first cultivating part included in the photobioreactor of the present invention.

Fig. 3 is a plan view illustrating an embodiment of the second cultivating part included in the tubular photobioreactor.

Fig. 4 is a schematic diagram illustrating an embodiment of an oxygen discharger coupled to the culture pipe.

Fig. 5 is a plan view illustrating an embodiment of a cultivating part having two first culture solution outlets.

### DETAILED DESCRIPTION OF THE INVENTION

In the following detailed description, only certain exemplary embodiments of the present invention are shown and described, by way of illustration. As those skilled in the art would recognize, the invention may be embodied in many different forms and should not be construed as being limited to the embodiments set forth herein. Also, in the context of the present application, when an element is referred to as being "on" another element, it can be directly on another element or be indirectly on another element with one or more intervening elements interposed therebetween. Like reference numerals designate like elements throughout the specification.

The present inventors performed various experiments in order to investigate the reason why cultivated photosynthetic microalgae attach on the inner surface of culture vessels. As a result, the two reasons are confirmed. The first one is recognized as the uncontrolled proliferation of microalgae along with the growth thereof according to applying periodical light condition and dark condition. The other is recognized that photosynthetic microalgae are coupled on the site where flow velocity is low because the flow of culture solution is restricted locally and then the site of attachment is expanded.

The present inventors tried to design a culture vessel capable of lowering proliferating rate of photosynthetic microalgae and securing proper flow of culture solution by applying only light conditions. However, the present inventors confirmed that it is impossible to obtain proper flow of culture solution even though raising stirring rate with a culture tank-type photobioreactor having an agitator.

Thus, the present inventors tried to run the culture solution containing photosynthetic microalgae using other methods rather than using only an agitator and confirmed that circulating the culture solution from the culture tank to a pipe whose two ends are coupled to the culture tank can make the culture solution flow well compared with the method using only an agitator. Accordingly, the present inventors came to invent a photobioreactor having a culture pipe with a light source and a pump in order to solve the above problems. The present inventors could run culture solution at a constant rate using the culture pipe and the pump of said photobioreactor and apply light conditions to the culture solution easily along with providing sufficient light to cultivate photosynthetic microalgae through the light source coupled to the culture pipe.

The photobioreactor according to embodiments of the present invention is described in detail with reference to the enclosed drawings hereinafter:

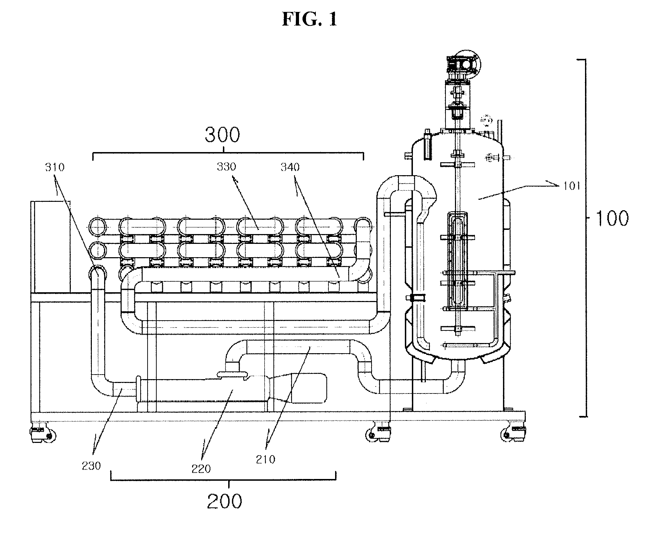

Fig. 1 is a schematic diagram showing a photobioreactor according to an embodiment of the present invention. As shown Fig. 1, the photobioreactor may comprise a first cultivating part 100, a pump part 200 and a second cultivating part 300. Photosynthetic microalgae are cultivated via circulation from the first cultivating part 100 to the pump part 200, from the pump part 200 to the second cultivating part 300 and then from the second cultivating part 300 to the first cultivating part 100, since the first cultivating part 100, the pump part 200 and the second cultivating part 300 are connected each other.

The second cultivating part 300 may be positioned apart from the first cultivating part 100. The first cultivating part 100 may be provided as a cylinder-type vessel as shown in Fig. 1, but the invention is not limited thereto. For example, the shape of the first cultivating part 100 can be of various forms such as a polygonal column. The second cultivating part 300 may be provided as a tubular shape and can include pipes molded with various shapes, etc. The pump part 200 is composed such that the culture solution can circulate between the first cultivating part 100 and the second cultivating part 300. For example, the pump part 200 can be positioned between the first cultivating part 100 and the second cultivating part 300 and can be equipped with a third culture solution inlet 210 connected to a first culture solution outlet 132 of the first cultivating part 100 and can be equipped with a third culture solution outlet 230 connected to a pump 220 and a second culture solution inlet 310 of the second cultivating part 300. Accordingly, the culture solution flowing from the first cultivating part 100 is transferred to the second cultivating part 300 and the culture solution circulates as follows: the first cultivating part 100 → the pump part 200 → the second cultivating part 300 → the first cultivating part 100. The pump part 200 can control the flow rate of culture solution circulating through the second cultivating part 300 via a flow rate controller coupled electronically thereto. In a preferred embodiment, the flow rate controller is built in the pump part 200. Control of the flow rate of culture solution is important for successful cultivation of Spirulina, because Spirulina is a multicellular spiral alga. If the flow rate is low, attachment of Spirulina occurs and gas exchange and illumination get worse due to inappropriate hydrodynamics. In contrast, high flow rate results in loss of useful materials in Spirulina. Thus, the flow rate should be controlled properly. It is preferred to control the flow rate between 1 to 50 cm/s. More preferably, the flow rate is 10 to 40 cm/s. In the most preferred embodiment, the flow rate is 20 to 30 cm/s.

In a preferred embodiment, the culture pipe has an inside diameter of 3 to 30 cm. In a more preferred embodiment, the inside diameter is 5 to 20 cm. In a further preferred embodiment, the inside diameter is 10 to 20 cm. In the most preferred embodiment, the inside diameter is 10 to 15 cm. The inside diameter of the culture pipe is important for cultivating Spirulina. If the inside diameter is more than 30 cm, the productivity gets worse due to inappropriate illumination. In contrast, if the inside diameter is less than 5 cm, it is difficult to scale-up the culture volume.

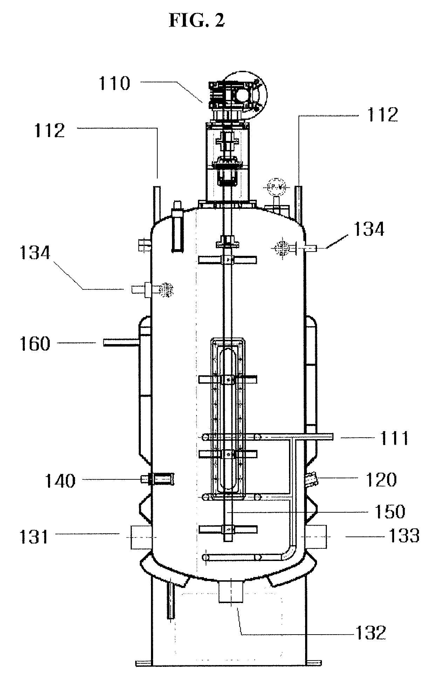

Fig. 2 is a sectional view of the first cultivating part 100 included in the photobioreactor of the present invention. As shown in Fig. 2, the first cultivating part 100 can be equipped with a cylindrical culture tank 101, an inoculum inlet 110, a gas inlet 111, a port for sensors 120, a first culture solution inlet 131, a first culture solution outlet 132, a final outlet 133, a pressure-controlling valve 112, a fresh media inlet 134, a first light source 140, an agitator 150 and a temperature-controller 160.

For example, the inoculum inlet 110 may be coupled to the upside of the culture tank 101, the gas inlet 111 and the port for sensors 120 may be coupled to the lower part of the culture tank 101. However, the arrangement is provided as an exemplary embodiment and may be varied according to changes of the shape of the culture tank 101.

The inoculum inlet 110 may be provided so as to inject various gases such as nitrogen and carbon dioxide gas mixture to the inside of the culture tank 101. Accordingly, it is possible to prevent contamination by various germs originating from the external environment during cultivating of photosynthetic microalgae by maintaining internal positive pressure in the culture tank 101. The positive pressure may be 0.1 to 1.0 kg/cM²f (10-100k Pa) as used for the conventional cultivation of microalgae, but not limited thereto.

The port for sensors 120 can be equipped with various sensors such as a pH sensor, a CO₂ sensor, a DO sensor and a temperature sensor.

In the meantime, the culture solution flows into the first cultivating part 100 from the second cultivating part through the first culture solution inlet 131 and flows out of the first cultivating part 100 to the pump part 200 via the first culture solution outlet 132. When the cultivation is finished, the final culture solution is released to the outside of the first cultivating part 100 through the final outlet 133.

In addition, the pressure-controlling valve 112 which is a one-way valve opened by the pressure of oxygen generated by cultivating the photosynthetic microalgae may be composed to discharge gases externally when the internal pressure is maintained as positive, but to be closed to stop discharging gases when the internal pressure is lowered.

The fresh media inlet 134 is a stick-type tube whose end is a globular shape, and may be provided as a spray ball shape having a plurality of micro nozzles on the surface of the globular end. Fresh media can be dispensed in a microspray into the first cultivating part 100 through the spray ball, thus the fresh media inlet 134 takes a role in eliminating foam generated in the first cultivating part 100. Further, the fresh media inlet 134 may be used for supplying detergents for washing the insides of the first cultivating part 100, the pump part 200 and the second cultivating part 300.

The first light source 140 which is a device for emitting light capable of facilitating photosynthesis during cultivation of photosynthetic microalgae emits light of three wavelengths or five wavelengths. In this regard, it is preferred that the intensity of illumination and the light-dark cycle are controlled automatically according to cultivation conditions. For example, the first light source 140 may be placed at the inside of the culture tank 101. In another embodiment, if the culture tank 100 comprises transparent materials or parts of the culture tank 100 is equipped with a transparent window through which light passes, the light source 140 may be placed at the outside of the culture tank 101.

The agitator 150 may be coupled to the inner-lower part and takes a role in mixing the culture solution in the primary culture, and mixing the culture solution remaining in the first cultivating part 100 with culture solution flowing from the first culture solution inlet 131. The temperature controller 160 may be coupled to the outside of the first cultivating part 100 and takes a role in controlling temperature. The temperature controller may be a water jacket capable of controlling culture temperature by circulating water with appropriate temperature through the jacket, but the invention is not limited thereto. Further, an observation window through which one can check the inside of the first cultivating part 100 may be installed additionally.

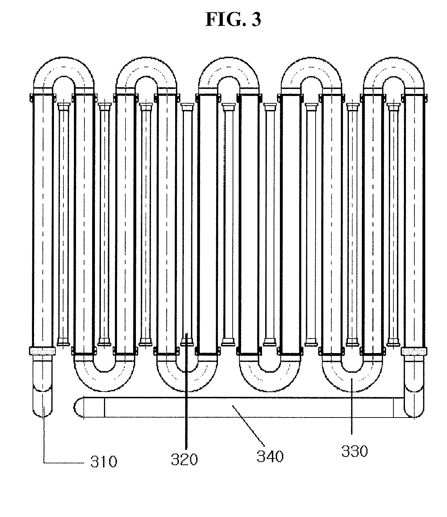

Fig. 3 is a plan view illustrating an embodiment of the second cultivating part 300 included in the tubular photobioreactor. As shown in Fig. 3, the second cultivating part 300 is equipped with a second culture solution inlet 310 connected to a third culture solution outlet 230 of the pump part 200, a culture pipe 330 and a second culture solution outlet 340 connected to the first culture solution inlet 131 of the first cultivating part 100.

The second light source 320 may be coupled to parts or whole of the second cultivating part 300. For example, the second light source 320 may be coupled to the outside of the culture pipe 330, extended longitudinally therethrough. The culture pipe 330 of the second cultivating part 300 plays a role in circulating the culture solution including photosynthetic microalgae and providing an environment where the circulating photosynthetic microalgae perform photosynthesis receiving light from the second light source 320. Thus, the culture pipe 330 may be composed of transparent materials in order to transmit the light emitted from the second light source 320. The whole culture pipe 330 may be composed of only transparent materials or only parts where light passes through are composed of transparent materials. Alternatively, the second light source 320 may be placed in the inside of the culture pipe 330. In this case, the second light source 310 is preferably coupled to the inner surface and is preferably an LED (light-emitting diode) and the culture pipe 330 may be composed of opaque materials. Additionally, sensors such as a pH sensor, a CO₂ sensor, a DO sensor, a temperature sensor may be coupled to one or more sides of the culture pipe 330. Further, the culture pipe 330 is preferably deformed as a narrow and long tubular shape in order to maximize area exposed to light. In this case, the culture pipe 330 is preferably deformed as a folded structure and the second light source 320 may be provided multiply between the folded structures of the culture pipe 330.

In an embodiment of the present invention, the culture pipe 330 of the second cultivating part 300 may be composed as a parallel multiple folded form including straight parts and curved parts on one frame or such a folded form may be piled up as a multi-layer structure. Also, the frame which is a scaffold for anchoring the second light source 320 may additionally comprise a power supply for the second light source 320. In addition, the second light source 320 plays a role in emitting light for the photosynthesis by the photosynthetic microalgae during the cultivation. Light emitted from the second light source 320 is preferably three-wavelength or five-wavelength similar to daylight but the invention is not limited thereto and it is preferred that the intensity of illumination and the light-dark cycle are controlled automatically according to cultivation conditions.

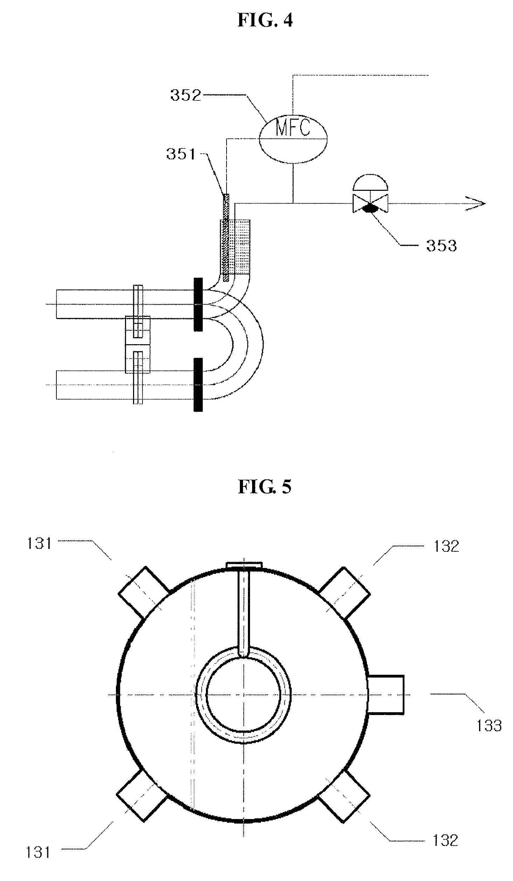

In the meantime, since the culture pipe 330 is deformed as a multiple folded structure including straight parts and curved parts, oxygen generated during cultivating photosynthetic microalgae may be accumulated in the curved parts without being discharged. In order to discharge the accumulated oxygen by force, it is preferable that the culture pipe 330 is additionally equipped with an oxygen discharger at the curved parts. When oxygen is accumulated above a certain level, the accumulated oxygen is discharged via automatic operation of the oxygen discharger (See Fig. 4).

Fig. 4 is a schematic diagram illustrating an embodiment of an oxygen discharger coupled to the culture pipe 330. As shown in Fig. 4, the oxygen discharger comprises an earth sensor 351, a mass flow controller 352 electrically connected to the earth sensor and a valve 353 connected the mass flow controller 352, and one end of the earth sensor 351 is immersed in the culture solution for applying an electric current.

If a gas layer is formed by the accumulation of oxygen in the curved parts, the earth sensor 351 is exposed from the culture solution and the electricity is interrupted. During the interruption, the valve 353 connected to the mass flow controller 352 is opened and then a certain amount of oxygen is expelled. When the earth sensor is re-immersed in the culture solution following the discharge of oxygen and the electric current is re-applied, the valve 353 closes.

In another aspect, the tubular photobioreactor of the present invention may include one cultivating part and two or more pump parts and culture pipe parts.

Fig. 5 is a plan view illustrating an embodiment of a cultivating part having two first culture solution outlet 132 providing culture solution with two pump parts, two first culture solution inlet 131 flowing in the culture solution from two culture pipes 330 and a cultivating part 100. As shown in Fig. 5, using the tubular photobioreactor comprising one cultivating part, two or more pump parts and culture pipe parts, the cultivating part provides the culture solution for the two culture pipe parts and the cultivation would be more efficient thereby.

Hereinafter, the action and the effect of the tubular photobioreactor are described in detail referring to the enclosed drawings:

First of all, the first culture solution inlet 131, the first culture solution outlet 132 and the final outlet 133 of the first cultivating part 100 are closed and an inoculum of microalgal seeds is injected to the culture tank 101 via the inoculum inlet 110 or fresh media inlet 134, And then, light is applied from the first light source 140 and a primary culture is performed while maintaining appropriate temperature using the temperature controller 160 and operating the agitator 150. The primary culture is terminated at an appropriate time after checking the culture condition using various sensors coupled to the port for sensors 120 of the first cultivating part 100.

And then, a fresh media is injected to the culture tank 101 via the fresh media inlet 134 and the agitator 150 is operated continuously for mixing the culture solution after the primary culture with the injected fresh media. When an equal volume of fresh media to the primary culture solution is injected to the culture tank 101, the first culture solution inlet 131 and the first culture solution outlet 132 are opened and the mixed culture solution is transferred to the third culture solution inlet 210 of the pump part 200. When the mixed culture solution is transferred to the third culture solution inlet 210 of the pump part 200, the pump 220 of the pump part 200 is operated and the mixed culture solution is transferred to the second culture solution inlet 310 of the second cultivating part 300 via the third culture solution outlet 230. The mixed culture solution transferred to the second culture solution inlet 310 of the second cultivating part 300 is transferred via the culture pipe 330, the second culture solution outlet 340 and the first culture solution inlet 131 of the first cultivating part 100, serially via the pump 220 and a circulation (the first cultivating part → the pump part → the second cultivating part → the first cultivating part) starts. When the circulation starts, light from the second light source 320 is emitted to illuminate the culture pipe 330 and photosynthesis by photosynthetic microalgae contained in the culture solution is carried out. Flow rate of the circulated culture solution may be controlled with a flow rate controller coupled electronically to the pump in order to prevent attachment of photosynthetic microalgae, especially Spirulina, and to maximize photosynthesis by circulating microalgae. The flow rate may be controlled between 1 to 50 cm/s. More preferably, the flow rate is 10 to 40 cm/s. In the most preferred embodiment, the flow rate is 20 to 30 cm/s, but not limited thereto.

During the circulatory cultivation of the photosynthetic microalgae, contamination by various germs originated from the environment is prevented by applying positive pressure of about 0.1 to 1.0 kg/cm²f (10-100 kPa) to the culture tank 101 of the first cultivating part 100 via injecting mixed gases of carbon dioxide and nitrogen to the culture tank 101 through the gas inlet 111 and the pH of the culture solution is controlled by adjustment of partial pressure of carbon dioxide included in the mixed gases.

In addition, oxygen generated by the photosynthesis during the cultivation of photosynthetic microalgae is transferred to the top of the culture tank 101 after being separated from the culture solution at the culture tank 101 of the first cultivating part 100 and then the oxygen is emitted externally through the pressure-controlling valve 112. In this case, if the culture pipe 330 of the second cultivating part 200 is composed as a parallel multiple folded form including straight parts and curved parts on one frame, oxygen may be accumulated at the curved parts of the culture pipe 330. Thus, it is preferred to remove oxygen accumulated in the culture pipe 330 by attaching one or more oxygen dischargers at the curved parts.

When the circulatory cultivation of photosynthetic microalgae is terminated, the final culture solution including the photosynthetic microalgae is harvested by opening the final outlet 133 of the first cultivating part 100. After the termination of the cultivation, the present inventors confirmed remarkable results that at most 5% of the inner surface of the culture pipe 330 is covered by the photosynthetic microalgae. Thus, the present inventors solved the problem that the efficiency of cultivation is lowered via attachment of photosynthetic microalgae to the inner surface of the culture vessel by lowering the rate of proliferation of photosynthetic microalgae through applying only light conditions using light sources equipped along the culture pipe and by maximizing fluidity of the culture solution via circulating the culture solution from the first cultivating part to the pump, from the pump to the second cultivating part and from the second cultivating part to the first cultivating part.

The photosynthetic microalgae may be genus *Chlorella,* genus *Gunaliella,* genus *Spirulina,* but not limited thereto. More preferentially, the photosynthetic microalgae is genus *Spirulina.*

While the present invention has been described in connection with certain exemplary embodiments, it is to be understood that the invention is not limited to the disclosed embodiments, but, on the contrary, is intended to cover various modifications and equivalent arrangements included within the scope of the invention, which is defined by the appended claims.

## Claims

1. A photobioreactor comprising the following:
a first cultivating part comprising a culture tank in which culture media are supplied, a first culture solution inlet and a first culture solution outlet each coupled to the culture tank, and a first light source coupled to the culture tank so as to illuminate the inside of the culture tank; and
a second cultivating part comprising a culture pipe arranged outside the culture tank in tubular shape and supplied with culture solution from the culture tank, a second culture solution inlet and a second culture solution outlet each coupled to the culture pipe, and a second light source coupled to the culture pipe so as to illuminate the inside of the culture pipe,
wherein the first culture solution outlet is connected to the second culture solution inlet and the first culture solution inlet is connected to the second culture solution outlet in order that the culture solution can be circulated between the first cultivating part and the second cultivating part.

2. The photobioreactor according to claim 1, further comprising a pump part connected to both the first cultivating part and the second cultivating part in order to circulate culture solution between the first cultivating part and the second cultivating part.

3. The photobioreactor according to claim 2, wherein the pump part comprises
a third culture solution inlet between the first culture solution outlet and the second culture solution inlet;
a third culture solution outlet between the first culture solution inlet and the second culture solution outlet; and
a flow rate controller which is electronically coupled to a pump of the pump part.

4. The photobioreactor according to claim 1, 2 or 3, wherein the first light source is placed inside the culture tank.

5. The photobioreactor according to any one of claims 1 to 4, wherein the second light source is coupled to the inner surface of the culture pipe or placed outside the culture pipe in the longitudinal direction of the culture pipe.

6. The photobioreactor according to any one of claims 1 to 5, wherein the culture pipe has a parallel multiple folded form and comprises a plurality of second light sources coupled to the outside of the culture pipe in the longitudinal direction of the culture pipe and/or has an inside diameter of 3 to 30 cm, preferably 10 to 20 cm.

7. The photobioreactor according to any one of claims 1 to 6, wherein the first cultivating part further comprises a fresh media inlet for supplying fresh media into the culture tank and a final outlet for releasing culture solution from the culture tank, wherein the fresh media inlet preferably has a spray ball shape including a globular type end and a plurality of micro nozzles on the globular type end.

8. The photobioreactor according to any one of claims 1 to 7, wherein the first cultivating part further comprises an agitator for mixing culture solution, which is coupled to the inside of the culture tank and/or wherein the first cultivating part further comprises a pressure-controlling valve for controlling pressure in the culture tank, wherein the pressure-controlling valve is preferably a one-way valve which opens by pressure of oxygen generated during the cultivation of photosynthetic microalgae.

9. The photobioreactor according to any one of claims 1 to 8, wherein the first cultivating part further comprises a temperature controller for controlling temperature in the culture tank, wherein the temperature controller is preferably a water-jacket coupled to the outside of the culture tank.

10. The photobioreactor according to any one of claims 1 to 9, wherein the first cultivating part further comprises one or more ports for sensors coupled to the culture tank and/or a gas inlet coupled to the culture tank so as to inject gases into the culture tank.

11. The photobioreactor according to any one of claims 1 to 10, wherein the second cultivating part further comprises an oxygen discharger, wherein the oxygen discharger preferably comprises an earth sensor, a mass flow controller electrically connected to the earth sensor and a valve connected to the mass flow controller; and
wherein the pump part further comprises a flow rate controller.
